# EUROPÄISCHE PATENTSCHRIFT

(11) **EP 2 240 148 B1**
(45) Veröffentlichungstag und Bekanntmachung des Hinweises auf die Patenterteilung: **14.11.2012**
(21) Anmeldenummer: 09710393.1
(22) Anmeldetag: 12.02.2009
(51) Int. Cl.: A61Q 19/10, A61K 8/02, A61Q 19/00

(54) **HYDROPHILES HAUTPFLEGETUCH**
HYDROPHILIC SKIN CLEANING CLOTH
LINGETTE HYDROPHILE POUR LA PEAU

(30) Priorität: 13.02.2008 CH 202082008
(43) Veröffentlichungstag der Anmeldung: 20.10.2010
(73) Patentinhaber: FILAG Schweiz AG, 8207 Schaffhausen (CH)
(72) Erfinder: MARTIN, Jürgen Anton, CH-8240 Thayngen (CH)
(74) Vertreter: Schneider Feldmann AG Patent- und Markenanwälte
(86) Internationale Anmeldenummer: PCT/EP2009/051651
(87) Internationale Veröffentlichungsnummer: WO 2009/101144

(56) Entgegenhaltungen:
- EP-A- 1 696 063
- GB-A- 2 384 789

## Beschreibung

### Technisches Gebiet

Die vorliegende Erfindung beschreibt ein hydrophiles einlagiges Hautpflegetuch, umfassend eine textile Fläche mit einer Gitterstruktur und Gitterzwischenplätzen, mit abrasiver Wirkung, welches eine mechanische Mikrodermabrasion ermöglicht und aus einem Gemisch aus hydrophilen und hydrophobem Material hergestellt ist.

### Stand der Technik

Reinigungstücher für die Haut, welche unter zur Hilfenahme von Wasser oder anderen mit Wirkstoffen versetzten Reinigungsflüssigkeiten vor allem zur Reinigung der Gesichtshaut eingesetzt werden sind seit langem bekannt.

GB2384789beschreibt ein Verfahren zur Herstellung eines Hautpflegetuchs mit einer Gitterstruktur und Gitterzwischenplätzen, die durch Weben oder Stricken einers Garns hergestellt wird. Die textile Fläche wird durch einen Splitting-Prozess aufgeraut, so dass mehrere Widerhaken entstehen.

Seit einiger Zeit sind auch Reinigungstücher, welche ein sogenanntes oberflächliches Peeling der obersten Hornschicht der Haut nur durch Anwendung mechanischer Mittel erlauben, bekannt. Dieses oberflächliche Peeling wird Mikrodermabrasion genannt, wobei die oberste Hornschicht der Haut von abgestorbenen Zellen befreit wird. Die benutzten Reinigungstücher bestehen meist aus Gewebe, welches aus einzelnen Mikrofasern mit Faserdurchmessern im Mikrometerbereich gewebt wurde. Das aus Mikrofasern hergestellte Gewebe der Reinigungstücher zeichnet sich durch eine grosse Oberfläche aus, wodurch Flüssigkeit extrem gut speicherbar ist und das Gewebe eine hohe Saugfähigkeit, was einem hohen Wasseraufnahmekoeffizient entspricht, aufweist. Durch immer kleinere Faserdurchmesser konnte in der Vergangenheit die Oberfläche noch erhöht werden, wodurch die Saugfähigkeit noch mal erhöht werden konnte. Die Optimierung des Mischungsverhältnisses aus hydrophilem und hydrophobem Material optimierte den Wasseraufnahmekoeffizient zusätzlich.

Damit die Mikrodermabrasion mit einem Reinigungstuch aus Mikrofaser möglich ist, wird das Gewebe nach dem Weben oder Wirken oder Stricken in einem sogenannten Splitting-Prozess gesplittet, wodurch die äusseren Schichten der einzelnen Mikrofasern angeraut werden und wiederhakenähnliche Strukturen entstehen, welche die Abrasivität des Gewebes erhöhen, wodurch Hautschichten abgetragen werden können. Durch den Splitting-Prozess wird ausserdern auch die Wasseraufnahmefähigkeit verbessert, da Wassermoleküle durch Widerhaken innerhalb des Gewebes festgehalten werden. Der Grad des Splitting-Prozesses beeinflusst neben der Flauschigkeit des Gewebes und der damit verbundenen Abrasivität auch die Wasseraufnahmefähigkeit.

Zur Pflege und zum Schutz der Haut können üblicherweise nach dem Peeling kosmetische oder pharmazeutische Wirkstoffe auf die Haut aufgebracht werden, wobei diese Zuführung von Wirkstoffen zeitlich nach der Reinigung und getrennt von der Reinigung erfolgt.

### Darstellung der Erfindung

Die vorliegende Erfindung hat sich zur Aufgabe gestellt ein Hautpflegetuch zu schaffen, mit welchem eine mechanische Mikrodermabrasion der obersten Hornschicht der Haut mit daran zeitlich direkt anschliessender lokaler Wirkstoffapplikation kombiniert erreichbar ist, wobei der Grad der Mikrodermabrasion der Haut und die Menge und die Art der auftragbaren Wirkstoffe durch das Hautpflegetuch vorgegeben ist.

Das erfindungsgemässe Hautpflegetuch ist einfach anwendbar und gewährleistet die Durchführung eines Peelings, wobei im gleichen Arbeitsgang Wirkstoffe auf die Haut aufgetragen werden, wobei nur das eine Hautpflegetuch eingesetzt wird.

In einer speziellen Ausführungsform wird ein kostengünstig herstellbares leichtes Hautpflegetuch beansprucht, welches als Einweg-Produkt für den Einweggebrauch hergestellt ist.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben.
- Figur 1a: zeigt eine schematische Ansicht eines eher zweidimensionalen Gewebes aus Mikrofasern, während
- Figur 1b: eine Detailansicht eines Ausschnittes des 2d-Gewebes der Figur 1a zeigt, wobei die Gitterstruktur und die Widerhaken deutlich werden.
- Figur 1c: zeigt ein Gewebe gemäss Figur 1b welches mit Wirkstoff im Wesentlichen innerhalb des 2d Gewebes benetzt ist.
- Figur 2a: zeigt eine schematische Ansicht eines dreidimensionalen, Schlaufen aufweisenden Gewebes aus Mikrofasern, während
- Figur 2b: eine Detailansicht des Gewebes aus Figur 2a zeigt, wobei durch Splitting verursachte Widerhaken an der Gitterstruktur und an den Schlaufen deutlich sind.
- Figur 2c: zeigt ein Gewebe gemäss Figur 2b welches mit einem Anteil mindestens eines Wirkstoffes benetzt ist.

### Beschreibung

Die Basis des vorliegenden erfindungsgemässen Hautpflegetuches bildet eine textile Fläche 1, welche gewoben, gewirkt oder gestrickt ist und welche auf Grund der verwendeten Materialien und/oder auf Grund der verwendeten Herstellungsmethode hydrophil ist und Wasser bzw. Flüssigkeiten in grosser Menge aufnehmen und festhalten kann. Diese Anforderungen erfüllt eine textile Fläche 1 aus Mikrofasern, welche eine Gitterstruktur 10 mit leeren Gitterzwischenplätzen 11 aufweist. Die verwendeten Mikrofasern weisen üblicherweise Durchmesser im Bereich kleiner 50 µm auf.

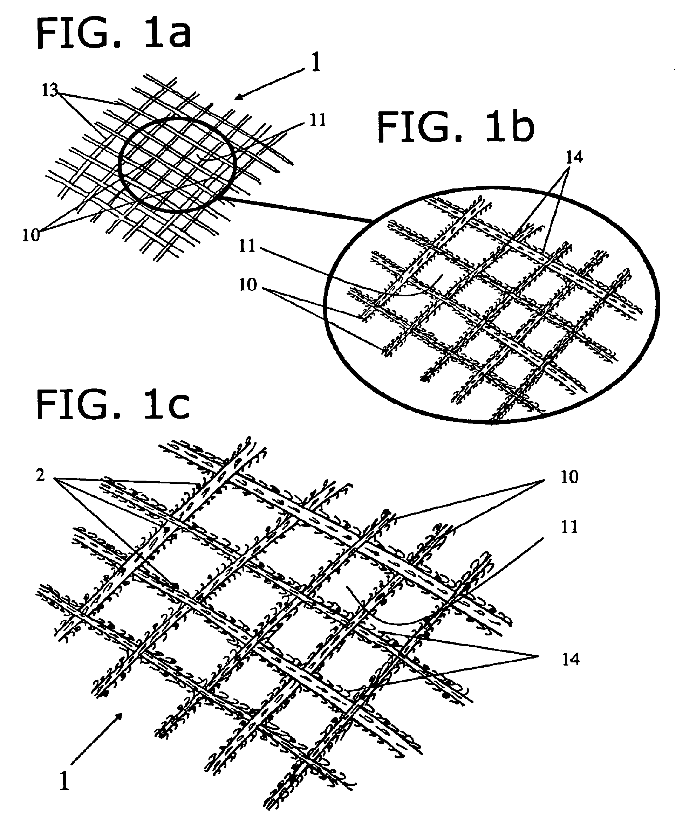

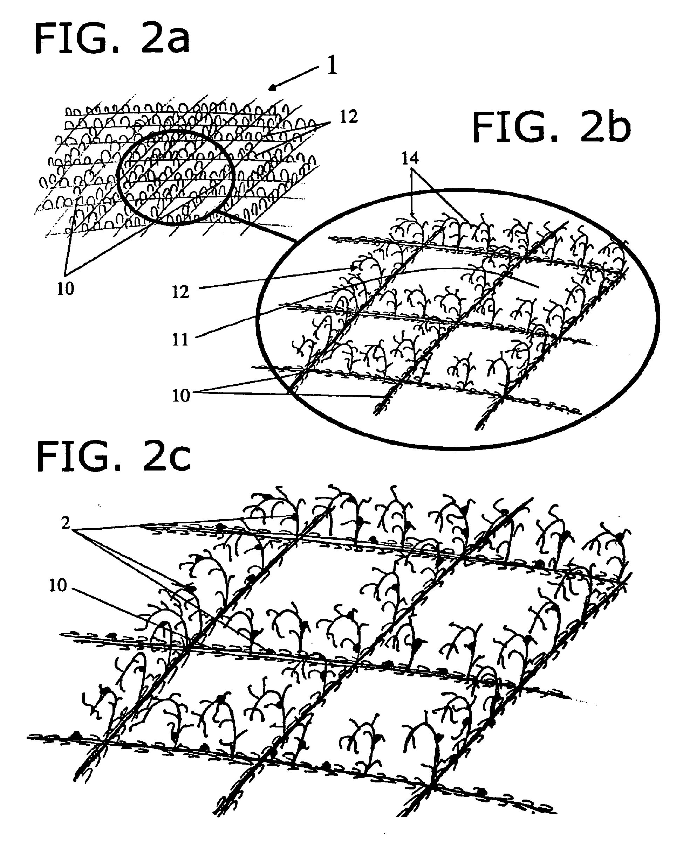

Die benutzte Webart und die daraus resultierende mikroskopische Gitterstruktur 10 der textilen Fläche 1 des Hautpflegetuches sind variabel. Ein Beispiel für eine eher zweidimensionale einlagige Gitterstruktur 10 der textilen Fläche 1 ist in Figur 1a gezeigt, während eine eher dreidimensionale Gitterstruktur 10 der textilen Fläche 1 mit annähernd senkrecht von der Ebene der Gitterstruktur 10 wegweisenden Schlaufen 12 in Figur 2a gezeigt ist.

Das aus Mikrofasern bestehende Hautpflegetuch weist einen hohen Wasseraufnahmekoeffizient auf. Der Wasseraufnahmekoeffizient ist ein Mass für die Wassersaugfähigkeit und gibt die Masse der Flüssigkeit, welche von einer Standardfläche pro Zeiteinheit aufgenommen wird, an. Durch kapillare oder absorptive Kräfte wird flüssigkeit von der textilen Fläche 1 aufgenommen und in der Gitterstruktur 10 gehalten. Dabei können sich Moleküle der Flüssigkeit in den Gitterzwischenplätzen 11 und/oder an den hydrophilen Mikrofasern 13 direkt anlagern.

Um die, für das erfindungsgemässe Hautpflegetuch nötige Abrasivität und einen möglichst hohen Wasseraufnahmekoeffizienten zu erreichen, wird die textile Fläche 1 nach dem Weben oder Wirken oder Stricken in einem Splitting-Prozess aufgeraut, wodurch die Oberfläche der textilen Fläche 1 noch deutlich erhöht werden kann. Der Splitting-Prozess wird üblicherweise durch ein Alkalibad der gewobenen, gestrickten oder gewirkten textilen Fläche 1 durchgeführt, wodurch der Mantel, welcher die einzelnen Mikrofasern 13 umgibt, teilweise gefasert gelöst wird. Der Splitting-Prozess führt zur Bildung von Widerhaken 14, wie sie in den Figuren 1b und 2b zu erkennen sind. Durch diese Widerhaken 14 wird neben der Oberfläche und der damit verbundenen Wasseraufnahmefähigkeit auch die Abrasivität der textilen Fläche 1 erhöht. Durch den Grad des Splitting-Prozesses kann die Abrasivität der textilen Fläche 1 beeinflusst werden, was wiederum die peeling-Eigenschaften bestimmt. Die Anzahl und die Länge der einzelnen Widerhaken 14 wird je nach Grad des Splitting-Prozesses ausgebildet.

Das erfindungsgemässe Hautpflegetuch ist bevorzugt aus einem Materialgemisch aus Polyamid und Polyester hergestellt. Versuche haben ergeben, dass für eine ausreichend hydrophile textile Fläche 1 ein Polyamidgehalt von grösser als 10%, bevorzugt grösser 25%, verwendet werden muss. Polyamid ist hydrophil, weshalb die Wasseraufnahmefähigkeit mit dem Polyamidgehalt korreliert ist. Sehr gute Ergebnisse wurden mit einem Polyamidgehalt von 40% und einem Polyestergehalt von 60% erreicht. Neben dem hydrophobem Polyester sind auch anderer hydrophobe Materialien verwendbar. Die textile Fläche **1** wird nach dem Weben oder Wirken oder Stricken in einem Splitting-Prozess so lange weiterbehandelt, bis ein Stück der textilen Fläche **1** mit einem gegebenen Eigengewicht eine Wassermenge in Höhe des mehrfachen des Eigengewichtes der textilen Fläche **1** aufnehmen kann. Besonders bevorzugt ist ein Hautpflegetuch, dessen textile Fläche **1** etwa das zwei- bis dreifache seines Eigengewichtes an Flüssigkeit aufnehmen kann.

Durch den Splitting-Prozess entsteht ein hydrophiles Hautpflegetuch mit abrasiver Wirkung, welches ein Flächengewicht, ein Gewicht pro Normfläche von einem Quadratmeter, von einigen hundert Gramm pro Quadratmeter aufweist. Da das erfindungsgemässe Hautpflegetuch bevorzugt als Einwegprodukt für den Einweggebrauch Anwendung findet, sind textile Flächen 1 mit einem Flächengewicht von kleiner als zweihundert Gramm pro Quadratmetern aus Kostengründen vorgesehen.

Nachdem die textile Fläche 1 des Hautpflegetuches in einem Splitting-Prozess veredelt wurde und die Widerhaken 14 von den Mikrofasern 13 wie in den Figuren 1b und 2b zu erkennen abstehen und teilweise in die Gitterzwischenplätze 11 hineinragen, wird mindestens ein Wirkstoff 2 in die textile Fläche **1** eingebracht. Diese Wirkstoffe 2 können wasserlöslich oder fettlöslich ausgeführt sein und aus der Klasse der pharmazeutischen oder der kosmetischen Wirkstoffe 2 stammen. Der Wirkstoff 2 wird mechanisch innerhalb der Gitterstruktur 10 an den Schlaufen 12, den Mikrofasern 13 an sich oder den Verästelungen der Widerhaken 14 gehalten. Innerhalb der textilen Fläche **1** wird eine definierte Masse reproduzierbarer Grösse des Wirkstoffes 2 eingelagert.

Bevorzugt ist eine Masse des Wirkstoffes innerhalb der textilen Fläche 1, welche etwa 2,5% bis 7,5% des Eigengewichtes der textilen Fläche 1 entspricht, insbesondere ist eine Masse des Wirkstoffes bevorzugt, welche etwa 2,5% bis 5% des Eigengewichtes der textilen Fläche **1** entspricht. Versuche haben gezeigt, dass je nach Anwendungsgebiet ein Hautpflegetuch mit einem Flächengewicht von 200 g/m² mit einer definierten Menge Wirkstoff 2 von 5 bis 10 g/m² für den Einweggebrauch gute Ergebnisse liefert. Mit einer solchen Beladung der textilen Fläche **1** kann bei einmaligem Gebrauch ausreichend viel Wirkstoff 2 auf die Haut eines Anwenders aufgebracht werden.

Die Wirkstoffe können auf verschiedene Arten in die textile Fläche **1** eingebracht werden. Beispielsweise kann der Wirkstoff in Wasser oder einem Lösungsmittel gelöst, oder in einer Wasser/Wirkstoff-Emulsion in einem Tauchbad vorliegen, durch welches die textile Fläche **1** hindurchgezogen wird. Einzelne Partikel des Wirkstoffes 2 verhaken sich bei diesem Prozess in der Gitterstruktur 10 und werden dort mechanisch angelagert. Diese Anbringung des Wirkstoffes 2 wird hier als mechanisch innerhalb der textilen Fläche **1** gehalten bezeichnet. Überschüssiges Wasser kann thermisch durch Verdunstung anschliessend entfernt werden.

Eine definierte konstante Masse mindestens eines Wirkstoffes 2 kann ausserdem, wie dem Durchschnittsfachmann bekannt, durch gezieltes Aufdampfen oder Aufsprayen einer Lösung des Wirkstoffes 2 in Wasser oder von einer Wasser/Wirkstoff-Emulsion auf die textile Fläche 1 aufgebracht werden.

Wenn der Wirkstoff 2 in Pulverform oder mikroverkapselt vorliegt, ist eine Bestäubung oder ein Beschuss der textilen Fläche 1 mit dem Wirkstoff 2 durchführbar, wobei eine kontrollierbare Menge des Wirkstoffes an der textilen Fläche 1 haften bleibt. Wenn der Wirkstoff 2 in Pulverform oder mikroverkapselt vorliegend auf die textile Fläche 1 gestäubt oder geschossen aufgebracht wurde, kann ein thermisches Verfahren zur Fixierung unterstützend angewendet werden, wodurch der Wirkstoff 2 thermisch an das Polyamid gebunden wird.

Die mit Wirkstoff 2 beaufschlagte textile Fläche 1 kann durch ein thermisches Verfahren nach dem Tauchbad, dem Aufdampfen oder dem Aufsprayen getrocknet werden, wobei Wassermoleküle aus der textilen Fläche 1 verdampfen und der reine Wirkstoff 2 zurückbleibt.

Das Einbringen einer Masse eines Wirkstoffes 2 in die textile Fläche 1 kann auch durch eine Pulverbeschichtung erreicht werden. Dabei ist es möglich den in Pulverform vorliegenden Wirkstoff 2 und/oder die textile Fläche 1 elektrostatisch aufzuladen und die textile Fläche 1 in geringem Abstand über dieses Pulver zu bewegen. Dabei wird der Wirkstoff 2 in Richtung der textilen Fläche 1 beschleunigt und dringt tief in die mit Widerhaken 14 verästelte der textile Fläche 1 ein und wird dort mechanisch innerhalb der textilen Fläche 1 gehalten. Ein anschliessender thermischer Prozess kann auch hier die dauerhafte Fixierung des Wirkstoffes 2 erhöhen.

Beispiele möglicher Wirkstoffe 2 sind Pflanzenöle, ätherische und/oder mineralische Öle, aber auch wasserlösliche pharmazeutische Wirkstoffe 2, welche adstringierend, durchblutungsfördernd, revitalisierend, entzündungshemmend, desinfizierend oder straffend wirken. Neben beispielsweise Sonnencreme für den UV-Schutz der Haut können kosmetische Wirkstoffe 2 eingesetzt werden, beispielsweise Bräunungsmittel oder Bleichmittel zum gezielten Tönen der Haut. Kräuterextrakte zur Beruhigung der Haut oder weitere Wirkstoffe 2, welche antiseptisch und/oder abschwellend und/oder wundheilend und/oder leicht kühlend und/oder juckreizlindernd wirken, sind von Interesse.

Der Anwender benutzt das Hautpflegetuch im Wesentlichen wie ein übliches Reinigungstuch des Stands der Technik, welches mit Wasser oder einer anderen Flüssigkeit benetzt wird und meist in kreisenden Bewegungen über die Gesichtshaut geführt wird. Aufgrund der Abrasivität des Hautpflegetuches werden überschüssige abgestorbene Hautzellen der obersten Hautschichten abgetragen. Durch die mechanische Verformung der textilen Fläche 1 bei der Bewegung des Hautpflegetuches, sowie durch das Einwirken der Flüssigkeit, werden die mechanisch gehaltenen Wirkstoffe 2 aus der textilen Fläche 1 von den Verästelungen der Widerhaken 14 gelöst, wodurch die Wirkstoffe 2 an die Haut gelangen und dort lokal aufgetragen werden.

Wenn der Wirkstoff 2 wasserunlöslich oder mikroverkapselt vorliegt, dann löst sich der Wirkstoff 2 zwangsläufig nicht in Wasser, so dass die Partikel des Wirkstoffes 2 längere Zeit brauchen, bis diese mechanisch aus der textilen Fläche 1 aus der Gitterstruktur 10, von den Gitterzwischenplätzen 11, von den Schlaufen 12, den Mikrofasern 13 oder Widerhaken 14 gelöst werden und an die Haut gelangen können. Da eine definierte Masse des Wirkstoffes 2 in der textilen Fläche 1 mechanisch gehalten ist, ist die korrekte und reproduzierbare Dosierung der Wirkstoffe 2 immer gegeben und eine Überdosierung ausgeschlossen.

Das erfindungsgemässe mit Wirkstoffen 2 versehene Hautpflegetuch der hydrophilen textilen Fläche 1 mit abrasiver Wirkung nimmt bei der Benutzung Feuchtigkeit auf, wodurch die Wirkstoffe 2 aus dem Inneren des Gewebes 1 lösbar sind. Nachdem die Wirkstoffe durch Wasserzugabe und Bewegung des Hautpflegetuches gelöst wurden, können die Wirkstoffe auf die Haut und in tiefer liegende Hautschichten aufgrund der vorhergegangenen peeling-Behandlung eingebracht werden. Aufgrund der definierten Menge des mechanisch gelagerten Wirkstoffes 2 erfolgt eine reproduzierbare Dosierung und lokale Auftragung der Wirkstoffe 2 auf die Haut. Neben dem Hautpflegetuch sind keine weiteren peeling-Vorrichtungen oder wirkstoffumfassenden Salben oder Cremes mehr nötig, weshalb eine kosmetische und/oder pharmazeutische Behandlung allein mit dem erfindungsgemässen Hautpflegetuch möglich ist.

In einer weiteren erfindungsgemässen Ausführungsform des Hautpflegetuches wird der Wirkstoff 2 in Form von Nanopartikeln mit Grössen von einigen hundert Nanometern bis zu einigen zehn Nanometern in das Gewebe 1 eingelagert.

Die bevorzugte Verwendung des Hautpflegetuches für den Einweggebrauch, verhindert, dass Schmutzpartikel, abgestorbene Hautzellen oder Mikroorganismen, welche auf bereits mehrfach verwendeten Hautpflegetüchern lagern, mit der Haut in Kontakt kommen.

Mikrofasertücher sind heute noch äusserst kostspielig, diese als Einwegtücher zu verwenden widerspricht dem gängigen kommerziellen Überlegungen. Durch die Verwendung von einlagigen Tüchern mit einem geringen Gewicht pro Flächeneinheit lässt sich der Kostenfaktor relativieren. Andererseits ist durch die hier interessierende Kombination von Mikrodermabrasion und Auftragen von Wirkstoffen 2 die Wiederbenutzbarkeit praktisch ausgeschlossen, da dies einen Waschvorgang bedingt, der die Wirkstoffe entfernt.

### Bezugszeichenliste

- 1: Textile Fläche
10 Gitterstruktur
11 Gitterzwischenplätze
12 Schlaufen
13 Mikrofaser
14 Widerhaken
- 2: Wirkstoff

## Patentansprüche

1. Hydrophiles einlagiges Hautpflegetuch, umfassend eine textile Fläche (1) mit einer Gitterstruktur (10) und Gitterzwischenplätzen (11), mit abrasiver Wirkung, welches eine mechanische Mikrodermabrasion ermöglicht und aus einem Gemisch aus hydrophilem und hydrophobem Material hergestellt ist, wobei die textile Fläche (1) aus Mikrofasern (13) besteht, welche einen Durchmesser von weniger als 50 µm aufweisen, in einem Splitting-Prozess aufgeraut sind und eine Mehrzahl von Widerhaken (14) aufweisen und die textile Fläche (1) mit einer definierten Menge mindestens eines Wirkstoffes (2), welcher mechanisch innerhalb der textilen Fläche (1) gehalten ist, beladen ist,
**dadurch gekennzeichnet, dass**
das Hautpflegetuch als Einweg-Produkt für den Einweggebrauch ausgeführt ist, indem die textile Fläche (1) des Hautpflegetuches ein Flächengewicht von weniger als zweihundert Gramm pro Quadratmeter aufweist, wobei der Wirkstoff (2) in Form von Nanopartikeln mit Grössen von einigen hundert Nanometern bis zu einigen zehn Nanometern in die textile Fläche (1) eingelagert ist und durch Flüssigkeitszufuhr und mechanische Verformung der textilen Fläche (1)
aus dem Inneren der textilen Fläche (1) freisetzbar ist und dadurch lokal auf die Haut auftragbar ist.

2. Hautpflegetuch nach Anspruch 1, **dadurch gekennzeichnet, dass** die textile Fläche (1) aus einem hydrophilen Polyamid/Polyester Gemisch mit einem Polyamidanteil von mindestens 10% hergestellt ist.

3. Hautpflegetuch nach Anspruch 1, **dadurch gekennzeichnet, dass** der Wirkstoff (2) wasserlöslich ist und sich durch die Benetzung des Hautpflegetuches mit Wasser löst.

4. Hautpflegetuch nach Anspruch 1, **dadurch gekennzeichnet, dass** der Wirkstoff (2) fettlöslich ist.

5. Hautpflegetuch nach Anspruch 4, **dadurch gekennzeichnet, dass** der mindestens eine Wirkstoff (2) Pflanzenöle und/oder ätherische Öle und/oder mineralische Öle umfasst.

6. Hautpflegetuch nach Anspruch 1, **dadurch gekennzeichnet, dass** der mindestens eine Wirkstoff (2) pharmazeutische Wirkstoffe (2) umfasst, welche adstringierend, durchblutungsfördernd, revitalisierend, entzündungshemmend, desinfizierend, abschwellend, wundheilend, kühlend, juckreizlindernd und/oder straffend auf die Haut wirken.

7. Hautpflegetuch nach Anspruch 1, **dadurch gekennzeichnet, dass** der mindestens eine Wirkstoff (2) ein Bräunungsmittel oder ein Bleichmittel zum gezielten Tönen der Haut umfasst.

8. Hautpflegetuch nach Anspruch 1, **dadurch gekennzeichnet, dass** der mindestens eine Wirkstoff (2) Kräuterextrakte umfasst.

9. Hautpflegetuch nach Anspruch 1, **dadurch gekennzeichnet, dass** der Wirkstoff (2) aufgesprayt, aufgedampft und/oder durch das Hindurchziehen durch ein Tauchbad in die textile Fläche (1) eingebracht ist.

10. Hautpflegetuch nach Anspruch 1, **dadurch gekennzeichnet, dass** die textile Fläche (1) etwa mindestens annähernd das Zweifache bis Dreifache seines Eigengewichtes an Wasser aufnehmen kann.

11. Hautpflegetuch nach Anspruch 1, **dadurch gekennzeichnet, dass** der Anteil des mindestens einen Wirkstoffes (2) innerhalb der textilen Fläche (1) weniger als 10% der Masse der textilen Fläche (1) entspricht.

12. Hautpflegetuch nach Anspruch 11, **dadurch gekennzeichnet, dass** der auf die textile Fläche (1) aufgebrachte Anteil des mindestens einen Wirkstoffes (2) etwa 2% bis 5% der Masse der textilen Fläche (1) entspricht.

## Claims

1. A hydrophilic single layer skincare cloth, comprising a textile surface (1) with a lattice structure (10) and intermediate spaces (11), with an abrasive action, which enables mechanical microdermabrasion, and is manufactured from a mixture of hydrophilic and hydrophobic material, wherein the textile surface (1) consists of microfibres (13), which have a diameter of less than 50 µm, are roughened in a splitting process, and have a multiplicity of barbed hooks (14), and the textile surface (1) is loaded with a defined quantity of at least one active substance (2), which is mechanically retained within the textile surface (1),
**characterised in that**,
the skincare cloth is embodied as a disposable product for single use, in which the textile surface (1) of the skincare cloth has a mass per unit area of less than two hundred grams per square metre, wherein the active substance (2) is stored in the textile surface (1) in the form of nanoparticles with sizes of between some hundreds of nanometres and some tens of nanometres, and by means of a supply of fluid and mechanical deformation of the textile surface (1) can be set free from the interior of the textile surface (1) and can thereby be locally applied onto the skin.

2. The skincare cloth in accordance with Claim 1, **characterised in that**, the textile surface (1) is manufactured from a hydrophilic polyamide/polyester mixture with a polyamide component of at least 10%.

3. The skincare cloth in accordance with Claim 1, **characterised in that**, the active substance (2) is water soluble, and is dissolved by wetting the skincare cloth with water.

4. The skincare cloth in accordance with Claim 1, **characterised in that**, the active substance (2) is fat-soluble.

5. The skincare cloth in accordance with Claim 4, **characterised in that**, the at least one active substance (2) comprises plant oils, and/or essential oils, and/or mineral oils.

6. The skincare cloth in accordance with Claim 1, **characterised in that**, the at least one active substance (2) comprises pharmaceutical substances (2) which act on the skin in a manner that is astringent, promotes circulation, is revitalising, anti-inflammatory, disinfecting, detumescing, heals wounds, is cooling, soothes itching, and/or is firming.

7. The skincare cloth in accordance with Claim 1, **characterised in that**, the at least one active substance (2) comprises a tanning agent or a whitening agent for specific skin tones.

8. The skincare cloth in accordance with Claim 1, **characterised in that**, the at least one active substance (2) comprises herbal extracts.

9. The skincare cloth in accordance with Claim 1, **characterised in that**, the active substance (2) is introduced into the textile surface (1) by means of a spray, vapour deposition, and/or by being passed through an immersion bath,

10. The skincare cloth in accordance with Claim 1, **characterised in that**, the textile surface (1) can hold approximately at least two or three times its own weight in water.

11. The skincare cloth in accordance with Claim 1, **characterised in that**, the proportion of the at least one active substance (2) within the textile surface (1) corresponds to less than 10% of the mass of the textile surface (1).

12. The skincare cloth in accordance with Claim 11, **characterised in that**, the proportion of the at least one active substance (2) applied onto the textile surface (1) corresponds to approximately 2% to 5% of the mass of the textile surface (1).

## Revendications

1. Lingette de soin pour la peau monocouche hydrophile, comprenant une surface textile (1) avec une structure de grille (10) et des emplacements intermédiaires de grille (11), avec effet abrasif, laquelle lingette permet une abrasion mécanique du microderme et est fabriquée à base d'un mélange de matériau hydrophile et hydrophobe, la surface textile (1) étant constituée de microfibres (13), qui présentent un diamètre de moins de 50 µm, sont rendues rugueuses dans un processus de "splitting" et présentent une pluralité de contre-crochets (14), et la surface textile (1) est chargée avec une quantité définie d'au moins un agent actif (2), lequel est maintenu mécaniquement à l'intérieur de la surface textile (1),
**caractérisée en ce que**
la lingette de soin pour la peau est réalisée sous forme de produit jetable pour l'usage unique, du fait que la surface textile (1) de la lingette de soin présente un poids surfacique de moins de deux cents grammes par mètre carré, l'agent actif (2) étant intégré dans la surface (1) textile sous forme de nanoparticules avec des grandeurs de quelques centaines de nanomètres jusqu'à quelques dixièmes de nanomètres et pouvant être libéré de l'intérieur de la surface textile (1) par arrivée de liquide et déformation mécanique de la surface textile (1) et pouvant être appliqué localement sur la peau.

2. Lingette de soin pour la peau selon la revendication 1, **caractérisée en ce que** la surface textile (1) est fabriquée à base d'un mélange polyamide/polyester hydrophile présentant une fraction de polyamide d'au moins 10 %.

3. Lingette de soin pour la peau selon la revendication 1, **caractérisée en ce que** l'agent actif (2) est soluble à l'eau et se détache par l'humectation de la lingette avec de l'eau.

4. Lingette de soin pour la peau selon la revendication 1, **caractérisée en ce que** l'agent actif (2) est soluble dans la graisse.

5. Lingette de soin pour la peau selon la revendication 4, **caractérisée en ce que** le au moins un agent actif (2) comprend des huiles végétales et/ou des huiles éthériques et/ou des huiles minérales.

6. Lingette de soin pour la peau selon la revendication 1, **caractérisée en ce que** le au moins un agent actif (2) comprend des agents actifs (2) pharmaceutiques, qui ont un effet astringent, favorisant l'irrigation, revitalisant, empêchant l'inflammation, désinfectant, dégonflant, cicatrisant, refroidissant, adoucissant pour les démangeaisons et/ou raffermissant sur la peau.

7. Lingette de soin pour la peau selon la revendication 1, **caractérisée en ce que** le au moins un agent actif (2) comprend un agent de brunissement ou un agent de blanchiment pour la coloration ciblée de la peau.

8. Lingette de soin pour la peau selon la revendication 1, **caractérisée en ce que** le au moins un agent actif (2) comprend des extraits de plantes.

9. Lingette de soin pour la peau selon la revendication 1, **caractérisée en ce que** l'agent actif (2) est appliqué par spray, vaporisé et/ou introduit dans la surface textile (1) par le passage à travers un bain d'immersion.

10. Lingette de soin pour la peau selon la revendication 1, **caractérisée en ce que** la surface textile (1) peut absorber à peu près au moins approximativement le double jusqu'au triple de son propre poids d'eau.

11. Lingette de soin pour la peau selon la revendication 1, **caractérisée en ce que** la fraction du au moins un agent actif (2) à l'intérieur de la surface (1) textile correspond à moins de 10 % de la masse de la surface (1) textile.

12. Lingette de soin pour la peau selon la revendication 11, **caractérisée en ce que** la fraction, appliquée sur la surface (1) textile, du au moins un agent actif (2) correspond environ à 2 % jusqu'à 5 % de la masse de la surface (1) textile.
